# EUROPEAN PATENT SPECIFICATION

(11) **EP 4 374 823 B1**
(45) Date of publication and mention of the grant of the patent: **26.08.2026**
(21) Application number: 22210053.9
(22) Date of filing: 28.11.2022
(51) Int. Cl.: A61M 25/09, A61F 2/01, A61F 2/95

(54) **AN INTRODUCER ASSEMBLY FOR MINIMALLY INVASIVE DEPLOYMENT OF A MEDICAL DEVICE IN A VESSEL**
EINFÜHRANORDNUNG FÜR MINIMALINVASIVEN EINSATZ EINER MEDIZINISCHEN VORRICHTUNG IN EINEM GEFÄSS
ENSEMBLE INTRODUCTEUR POUR DÉPLOIEMENT MINIMALEMENT INVASIF D'UN DISPOSITIF MÉDICAL DANS UN VAISSEAU

(43) Date of publication of application: 29.05.2024
(73) Proprietor: Emboline, Inc., Santa Cruz, CA 95060 (US)
(72) Inventor: JÖNSSON, Anders, 16757 Bromma (SE)
(74) Representative: KIPA AB

(56) References cited:
- WO-A1-2013/142386
- US-A1- 2002 161 389
- US-A1- 2005 209 582
- US-B1- 6 214 026
- US-B1- 6 290 656

## Description

### TECHNICAL FIELD

This disclosure pertains in general to introducer assemblies for minimally invasive deployment of a medical device in a vessel, e.g. cerebral embolic protection assemblies to prevent emboli from entering arteries branching from the aorta, e.g., arteries that lead to the brain. In particular, the disclosure relates to improvements of cerebral embolic protection assemblies.

### BACKGROUND

Particles such as emboli may form, for example, as a result of the presence of particulate matter in the bloodstream. Particulate matter may originate from for example a blood clot occurring in the heart. The particulate may be a foreign body, but may also be derived from body tissues. For example, atherosclerosis, or hardening of the blood vessels from fatty and calcified deposits, may cause particulate emboli to form. Moreover, clots can form on the luminal surface of the atheroma, as platelets, fibrin, red blood cells and activated clotting factors may adhere to the surface of blood vessels to form a clot.

Various medical procedures may perturb blood vessels or surrounding tissues. When this occurs, potentially harmful particulates, such as emboli, may be released into the blood stream. These particulates can be damaging, e.g., if they restrict blood flow to the brain. Since emboli are typically particulate in nature, various types of filters have been proposed in an attempt to remove or divert such particles from the bloodstream before they can cause damage to bodily tissues. As intravascular devices and procedures, such as Transcatheter Aortic Valve Implantation (TAVI), become more advanced, there is an emerging need for features that provide these devices with improved ease of use, intravascular stability, and embolic protection. Devices to block or divert particulates from flowing into particular regions of the vasculature have been proposed but may not eliminate the risks associated with the release of potentially harmful particulates into the blood stream during or after particular medical procedures.

Various systems for capturing embolic material during intravascular interventions are known. WO 2013/142386 A1 discloses a guidewire-mounted filter deployable distal of a treatment site for collecting embolic debris, with controlled expansion and retraction means. US 2002/0161389 A1 describes deployment and recovery control systems including a guidewire with an expandable filter, a restraining sheath, a torque control device and coaxial recovery components for collapsing and retrieving the filter. US 6 214 026 B1 discloses a vascular device with an articulation region and a support hoop carrying a blood-permeable filter, the articulation improving flexibility and delivery. US 2005/0209582 A1 relates to a multi-lumen catheter having separate lumens for guidewire passage, fluid delivery and aspiration, reinforced to enhance flexibility and prevent kinking. US 6 290 656 B1 discloses a guidewire-mounted expandable filter and coaxial sheath system for deployment and retrieval of an embolic protection device configured to retain captured debris during withdrawal.

Possible areas of improvements of such devices and procedures include prevention of "windsailing" of devices with pulsatile blood flow, leakage of fluid and/or particulate matter at peripheral portions of devices during use thereof, secure positioning in a patient during use and/or retrievability, etc.

Hence, an improved intravascular device, system and/or method would be advantageous and in particular allowing for increased flexibility, cost-effectiveness, and/or patient safety would be advantageous.

An object of the invention is to define a cerebral embolic protection assembly that further increases patient safety.

### SUMMARY

Accordingly, embodiments of the present invention preferably seek to mitigate, alleviate or eliminate one or more deficiencies, disadvantages or issues in the art, such as the above-identified, singly or in any combination by providing an introducer assembly according to the appended patent claims. The present invention is defined by the appended claims only, in particular by the scope of appended independent claim(s). Reference(s) to "embodiments" throughout the description which are not under the scope of the appended claims merely represents possible exemplary executions and are therefore not part of the present invention.

The aforementioned objects are achieved according to the disclosure.

In a broad aspect of the invention, an introducer assembly is provided for minimally invasive deployment of a medical device in a vessel. The assembly generally includes a handle, and a locking mechanism for locking a guidewire relative said handle.

The locking mechanism is configured to, in use, be able to positionally lock a guidewire in relation to the handle. In some embodiments the handle comprises the locking mechanism. In further embodiments the locking mechanism of the embolic protection assembly comprises a dampening function.

A primary purpose is to provide a means to be able to perform an operation with improved safety when using a cerebral embolic protection assembly. This is obtained by having a locking mechanism positionally locking a guidewire, thus avoiding to manually hold the guidewire. Other advantages will become apparent from the detailed description.

### BRIEF DESCRIPTION OF THE DRAWINGS

Some examples will now be described in more detail for explanatory, and in no sense limiting, purposes, with reference to the following figures, in which
- Fig. 1: illustrates an embolic protection assembly 100 laid out flat in a deployed state;
- Fig. 2a,2b: illustrate deployed embolic protection assemblies 201 according to the invention in use;
- Fig. 3: illustrates schematically an example of a basic locking mechanism according to the disclosure;
- Fig. 4: illustrates schematically an example of a combined locking and dampening mechanism according to a further aspect of the invention;
- Fig. 5: illustrates schematically an example of a combined locking and dampening mechanism with a controllable release 490 according to a further aspect of the invention.

### DETAILED DESCRIPTION

Specific embodiments of the invention will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

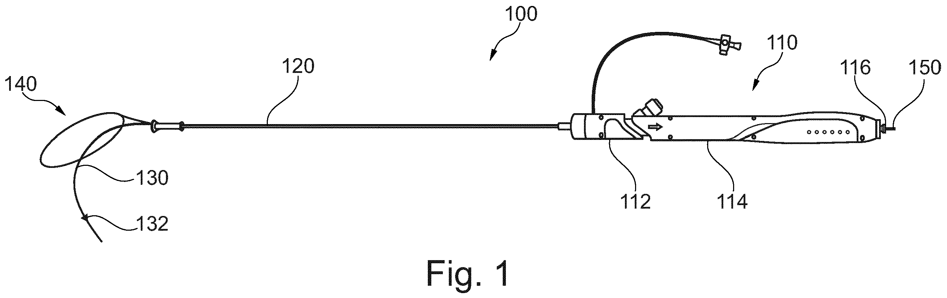

In order to clarify the assembly according to some examples and of their use will now be described in connection with Figures 1 to 5.

The present disclosure focuses on examples of the present invention defining an improved introducer assembly, such as an embolic protection assembly for example comprising an embolic protection device with a deflection-filter. The embolic protection device, suitably a collapsible embolic protection device, is in examples configured for delivery to an aortic arch of a patient and keeping it there in place for protection of side branch vessels of the aortic arch from embolic material. In particular, an embolic protection device having a deflection-filter membrane with a two- or three-dimensional structure. The embolic protection device is preferably delivered transvascularly, for instance in a trans-femoral approach. In some alternative examples, the device may be configured to be delivered through side branch vessels of the aortic arch.

Examples of embolic protection devices are disclosed in WO 2019/081689 A1 or WO 2018/206160A1.

Fig. 1 illustrates an example of an embolic protection assembly 100 laid out flat in a deployed state to be able to visualize all the different parts and how they are interconnected. The embolic protection assembly 100 comprises a handle 110 for delivery, deployment and retrieval. The handle 110 in the illustrated example is a two part handle. The handle 110 is shown in the deployed state with the two parts of the handle pushed together, in a deployed state an embolic protection device 140 is pushed out of a delivery system 120. For retrieval, the two parts of the handle 110 are pulled apart, the embolic protection device 140 is pulled into the delivery system 120. A distal part 112 of the handle 110 is connected to the delivery system 120, suitably a sheath or catheter. A proximal part 114 of the handle 110 is connected to a proximal end of a hypotube 130. The hypotube 130 can suitably be a nitinol curved hypotube shaft. The curvature of the hypotube 130 is configured to press the embolic protection device 140 comprising a deflection-filter 142 into place when deployed, and then keeping it in place until it is retrieved.

The deflection-filter 142 membrane is arranged to separate a first fluid volume of the aortic side branch vessels from a second fluid volume in the aortic arch when the protection unit is positioned in the aortic arch and maintained by a hypotube. The deflection-filter 142 membrane may be a 3-dimensional structure, such as a dome-shape. This may create space underneath the embolic protection device 140 and also may provide optimized filtering due to a larger filter area in relation to a flat or near flat deflection-filter 142 membrane.

The embolic protection device 140 can for example suitably comprise a self-positioning nitinol support frame without stabilizers with a PEEK mesh as a deflection-filter 142 or for example a nitinol frame with stabilizers and a nitinol mesh.

The deflection-filter 142 may suitably be made of an elastic and atraumatic material such as a polymer, e.g. a mesh made from a plurality of fibers made of polymer, nylon, nitinol, or metal, or a combination thereof. The mesh may be made from woven fibers. Fibers may be from about 20 to 50 µm in thickness. Alternatively, the deflection-filter 142 may be a perforated film. When a perforated film is present, the pores formed in the perforated film may include pores of varied or unvaried shape (e.g., rectilinear or rhomboid pores), have a varied or constant density across the film, and/or have a constant or varied size. The size of the pores of the filter allows passage of blood cells (e.g., red blood cells (erythrocytes), white blood cells (leukocytes), and/or platelets (thrombocytes)) and plasma, while being impermeable to particles (e.g., emboli) larger than the pore dimensions. Emboli filtered by the mesh of the filter of the present disclosure are typically particles larger in one or more dimensions than an aperture of the mesh of the filter. The deflection-filter 142 is further advantageously configured for collapsibility and/or crimpability of the embolic protection device 140.

The deflection-filter 142 of the embolic protection device is coupled to a distal part of the hypotube 130 via a device-tail/connection-mechanism 144 with a connector 146. The connection-mechanism 144 may in some embodiments have an integrated connector 146 and/or an integrated coupling to the deflection-filter. In other embodiments the connector 146 and/or the coupling to the deflection-filter 140 may be at least partly comprised of separate parts in relation to the connection-mechanism 144. In some embodiments the coupling to the deflection-filter may pivot. The connection-mechanism 144 can for example be a twisted wire suitably made of nitinol for a flexible connection-mechanism 144. Alternatively, the connection-mechanism 144 may be more or less rigid, for example made from a laser cut tube.

At a proximal end of the proximal part of the handle 114, there is a port 116 for a guide wire 150. A proximal part of the guidewire 150 will stick out here during use. A distal end of the hypotube 130 suitably comprises an atraumatic tip 132, from which a distal part of the guidewire 150 appears during use.

During use of this embolic protection assembly 100, after deployment of the embolic protection device there will be one person holding the handle 110 to make sure that the hypotube 130 is pushed up towards the deflection-filter 140 with a suitable force and making sure that it is in the correct place. A further person will ensure that the guidewire 150 is kept in place.

To improve ease of use and relieve one person or at least one hand of one person to hold the guidewire in place, according to the invention a locking mechanism is applied to the guidewire to replace manually holding the guidewire. The locking mechanism should preferably be activated to lock a guidewire first when the embolic protection device is deployed and in place, and unlocked when readjustment is necessary. The locking mechanism movably locks the guidewire in relation to an entry point into the body or in relation to the handle, or in relation to a catheter or sheath in which the guidewire is running.

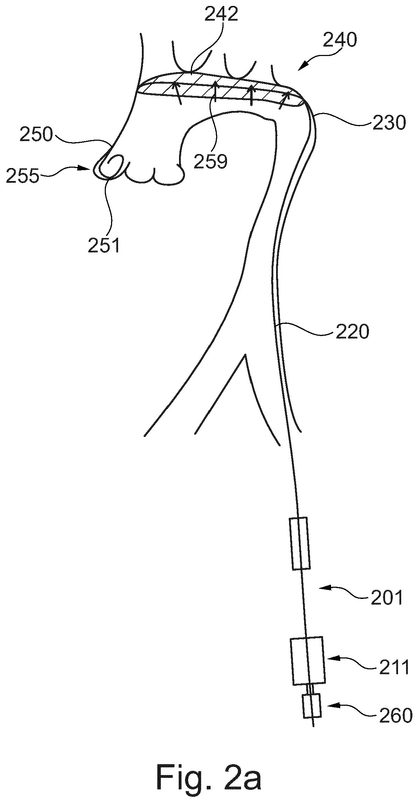

Figure 2a illustrates a simplified deployed embolic protection assembly 201 according to the invention in use. The embolic protection assembly 201 comprises a handle 211, a delivery system 220, a hypotube 230, an embolic protection device 240 comprising a deflection/filter 242, a guidewire 250 with a pigtail distal end 251, and a locking mechanism 260. The guidewire 250 runs through the hypotube 230, which in turn goes underneath the deflection/filter 242 of the embolic protection device 240. The locking mechanism 260 positionally locks the guidewire 250 for example in relation to the handle 211. The distal end 251 of the guidewire rests against a valve 255 which enables the guidewire to be pushed against the valve forcing the hypotube 230 to press up 259 against the deflection/filter 242. Thus, by regulating under what pressure the guidewire 250 is positionally locked, it is possible to regulate the force with which the hypotube is pressing up against the deflection/filter 242.

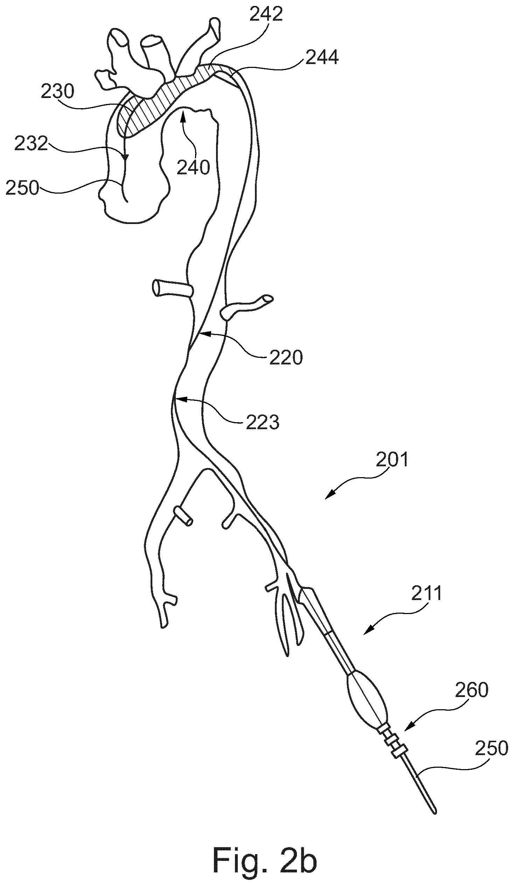

Figure 2b illustrates an in use deployed embolic protection assembly 201 according to the invention. The embolic protection assembly 201 comprises a handle 211, a delivery system 220, a hypotube 230, and an embolic protection device 240. The embolic protection device 240 in turn comprises a deflection filter 242 with a connection-mechanism 244 coupled to a hypotube 230. The hypotube 230 suitably comprises an atraumatic tip 232 at its distal end. An example of a locking mechanism 260 of a guidewire 250 according to the invention is illustrated integrated with the handle at a proximal end of the handle 211. The embolic protection assembly is illustrated in place in the aortic arch and the embolic protection device 240 fully deployed. The embolic protection device is suitably a self-aligning and self-expanding 3-dimensional structure that will line the inner wall of the aortic arch. The embolic protection device suitably spans over the whole apex from the ascending aorta to the descending aorta of the aortic arch preventing emboli from entering any of the side branches, which are supplying blood to the brain. A large filtering area will provide good filtering and provide a better sealing against the walls of the aortic arch. As can be seen in the figure, the delivery system 220 has a forced arch 223 indicating that the handle with a positionally locked guidewire is being pressed towards the aortic arch, resulting in the hypotube 230 being pressed up towards the deflection filter 242, thus keeping the embolic protection device 240 sealed in place during a complete heart cycle.

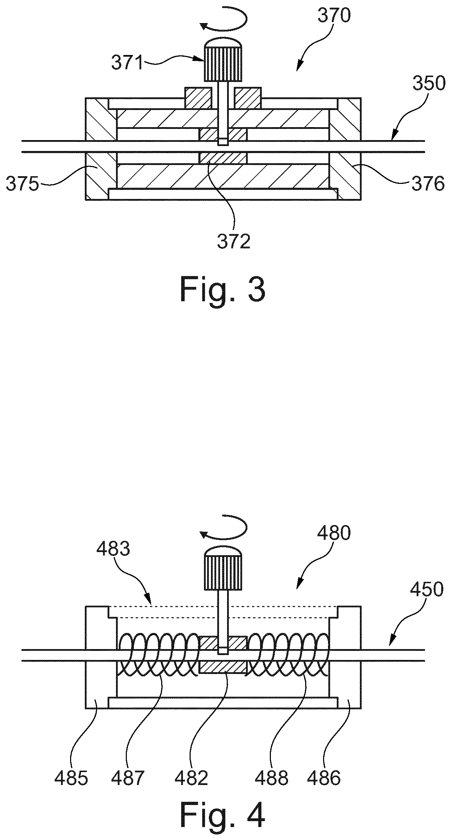

The locking mechanism 260 is a part of the handle 211. The locking mechanism 260 may comprise a dampening spring function especially when the locking mechanism is incorporated with the handle 211. The dampening spring function is further described below in relation to figure 4. A separately applicable locking mechanism may comprise a bodily fixed fastening device, such as a strap to encompass a bodily part or limb. The bodily fixed fastening device suitably comprises means for the locking mechanism to positionally lock the guidewire in relation to the bodily fixed fastening device. The main purpose of the locking mechanism of the guidewire is to be able to need/require less manual assistance. This relieves personnel from this task enabling them to work in a safer and possibly quicker manner. The locking mechanism locks the position of the guidewire in relation to one or more of a handle, an introducer, a sheath, a catheter, a body part, or an entry point.

Figure 3 illustrates schematically an example of a basic locking mechanism 370 according to the disclosure. It comprises a clamping box 372 that is movably fixed in relation to a first end 375 and/or a second end 376 of the basic locking mechanism 370. A guidewire 350 to be positionally locked runs through the clamping box 372 and is positionally locked in relation to the clamping box 372 by means of a screw 371 that when screwed into the clamping box 372 will clamp a guidewire 350 to the clamping box 372. A guidewire 350 that is positionally locked to the clamping box 372 will be released and positionally unlocked by un-screwing the screw 371. The basic locking mechanism 370 may be an integral part of a handle, for example such as previously described. If the locking mechanism is an integral part of a handle then the clamping box of the locking mechanism can be positionally/movably fixed in relation to a delivery system, a sheath, a catheter, a hypotube, or the handle itself. Alternatively, the basic locking mechanism 370 may be positionally attached to a body part, for example by means of a strap around a limb.

The clamping of a guidewire in a clamping box may be frictionally limited to allow a guide wire to move in relation to the clamping box if the guidewire is subjected to a force in relation to the clamping box that is above a first predetermined level. Alternatively, or additionally the locking mechanism may comprise a release mechanism, that releases a guidewire that is in a locked state into an unlocked state, when a guidewire is subjected to a force in relation to the clamping box that is above a second predetermined level. These additions are to prevent bodily harm from a distal end of a guidewire when a guidewire is in a locked state and the handle is moved intentionally or by accident.

Figure 4 illustrates schematically an example of a combined locking and dampening mechanism 480 according to an aspect of the invention. A dampening mechanism according to this illustrated embodiment is integrated with the locking mechanism. The dampening mechanism is to prevent bodily harm from a distal end of a guidewire when a guidewire is in a positionally locked state and the handle is moved intentionally or by accident.

Just like the locking mechanism according to figure 3, the dampening locking mechanism 480 according to figure 4 comprises a clamping box 482, a first end 485 and a second end 486. A guidewire 450 to be positionally locked running through the clamping box 482 and is positionally locked in relation to the clamping box 482 by means of a screw 471 that when screwed into the clamping box 482, the clamping box 482 will clamp a guidewire 450 to the clamping box 482. A guidewire 450 positionally locked to the clamping box 482 will be released and positionally unlocked by un-screwing the screw 471.

In contrast to the embodiment of figure 3, the clamping box 482 of the embodiment according to figure 4 is neither positionally locked in relation to the first end 485, nor in relation to the second end 486. The clamping box 482 is moveable between the two ends 485, 486 and a protective housing would then suitably have a slot 483 for the screw 471. In a preferred version of the embodiment, the movement of the clamping box 482 is dampened by at least one spring or spring function between the clamping box 482 and one of the ends 485, 486. Preferably there are two springs and/or spring functions, a first spring 487 between the clamping box 482 and the first end 485 and a second spring 488 between the clamping box 482 and the second end 486. The springs 487, 488 can be of a linear type or be of a progressive type. A linear spring has a spring rate that is consistent along the entire length of the spring as it is compressed. On the other hand, a progressive spring has a spring rate that increases or changes with the compression of the spring.

The two springs 487, 488 may be the same length and type, which would then position the clamping box 482 in the middle between the first end 485 and the second end 486 when in rest. This would result in a symmetrical dampening locking mechanism 480, which is suitable for a dampening mechanism 480 that is not integrated with a handle or does not comprise a connection element for attachment on only one correct/intended side, or for example keyed attachment connection elements. A symmetrical dampening locking mechanism cannot be turned in the wrong direction, working equally well in both directions. Preferably with an integrated or with a keyed attachment connection element to a handle, the dampening locking mechanism is asymmetrical having a longer stroke toward a proximal end of the dampening locking mechanism than a distal end towards a distal end of a guidewire. This is a safety mechanism to avoid the distal end of a guidewire being pushed into somewhere that could cause bodily harm.

In addition, as also described as an addition for the embodiment according to figure 3, the clamping of a guidewire 450 in a clamping box 482 may be frictionally limited to allow a guidewire to move in relation to the clamping box if the guidewire is subjected to a force in relation to the clamping box above a first predetermined level. Alternatively, or additionally the locking mechanism may also comprise a release mechanism, that releases a guidewire that is in a locked state into an unlocked state, when a guidewire in relation to the clamping box is subjected to a force above a second predetermined level. These additions are to further prevent bodily harm from the distal end of a guidewire when a clamping box 482 has by displacement reached one of the two ends 485, 486.

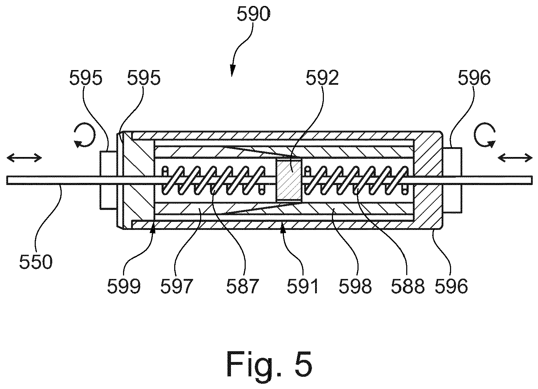

Figure 5 illustrates schematically an example of a combined locking and dampening mechanism with a controllable release 590 according to a further aspect of the invention. The locking mechanism 590 comprises a first end 595 and a second end 596, a lock unit 592 therebetween with a spring 587, 588 on either side. The locking mechanism 590 according to the example is configured for a guidewire 550 to run between the two ends, through the two springs, and through the lock unit. Initially the lock unit will be located centrally in between the two springs, assuming that the two springs have the same length and the same spring characteristics. As the two ends are turned such that they come closer together, first a guidewire through the lock unit becomes positionally locked to the lock unit and then the lock unit will also become positionally locked within the locking mechanism with an increasing friction as long as the two ends are continued to be turned to move the two ends closer together. A force on the guidewire in relation to the locking mechanism that overcomes the friction will move the lock unit out of the locking region and releasing both the lock unit and the guidewire. Suitably the maximum friction attainable is such that a force on the guidewire in relation to the locking mechanism required to release the guidewire is less than a force that can cause damage by the guidewire. Turning the two ends in the opposite direction in relation to each other will then first decrease the friction with which the lock unit is positionally locked within the locking mechanism until both the lock unit and a guidewire through it will become positionally unlocked.

In the illustrated example the second end 596 comprises a tubular external extension 591 that reaches the first end 595 where the first end 595 and the extension 591 are attached with threads. By turning the first end 595 and the second end 596 in relation to each other, the first end 595 and the second end 595 will either move apart or move closer together depending on the threads and how the ends 595, 596 are turned in relation to each other. Internally the first end 595 is coupled to a first tubular extension 597 with a distal end that is externally tapered, and the second end 596 is coupled to a second tubular extension 598 with a distal end that is internally tapered. The tapered end of the first extension is inside the tapered end of the second extension, and at this place inside the first extension is where the lock unit is located. When the ends are turned such that the two ends come closer together then the lock unit becomes more and more squeezed positionally locking the guidewire to the lock unit. At the same time the friction also increases between the lock unit and the inside of the distal end of the first tubular extension. This increases a necessary force on the guide wire in relation to the locking mechanism before the lock unit will move. Once the lock unit moves out of the squeezed region, the guidewire becomes positionally unlocked from the lock unit.

To improve the function of the embolic protection device even further, a filter cleaning mechanism can be incorporated in cooperation with the filter. A particle might stick to the filter or get stuck in a crease of the embolic protection device. These potential sticky or stuck particle(s) might become dislodged during removal of the embolic protection device and flow somewhere unwanted. The cleaning mechanism may comprise one or more of:
- A scraper attached to an optional (guide-) wire that is dragged and possible pushed back and forth over the filter of the embolic protection device.
- A mechanical vibration of the hypotube attached to the embolic protection device and or to a guidewire within the hypotube. Ultrasonic and or vibration waves sent along the hypotube and/or a guidewire within the hypotube. There can alternatively be external ultrasonic or vibration stimulus. Can also be lower or higher frequencies.
- A shaking mechanism of the guidewire.

To improve this cleaning mechanism even further, it is beneficial if the cleaning mechanism function is synchronized with the heartbeat, for example by means of EKG. Suitably the function is initiated at the beginning of the ventricular ejection and stopped before the ventricular ejection ends (ventricular systole, second phase). This to ensure that any particles that are dislodged by the cleaning mechanism are transported away from the area of the embolic protection device.

To improve the function of the embolic protection device still further, an embolic protection device removal synchronizer function can be incorporated in cooperation with the embolic protection device. As mentioned above, a particle might stick to the filter or get stuck in a crease of the embolic protection device. These potential sticky or stuck particle(s) might dislodge during removal of the embolic protection device and flow somewhere unwanted. The embolic protection device removal synchronizer function synchronizes the removal of the embolic protection device with the heartbeat, suitably by means of EKG or other suitable means. The removal of the embolic protection device should suitably be done during the interval when the blood pressure starts to increase since anything that is then dislodged will be flushed downstream. Otherwise, dislodged particles might just instead float about when the embolic protection device is folded and pulled away and then be flow up the previously filtered entrances when the pressure returns. A removal of an embolic protection device from its place of protection might thus take several heartbeats to complete.

The invention is based on the basic inventive idea of keeping an embolic protection device safely in place without less continuous manual intervention. The invention is not restricted to the above-described embodiments, but may be varied within the scope of the appended claims.
Figure 1 illustrates an embolic protection assembly laid out flat in a deployed state.
   - 100: An embolic protection assembly
   - 110: A handle
   - 112: A distal part of the handle
   - 114: A proximal part of the handle
   - 116: A guidewire port at a proximal end of the proximal part of the handle
   - 120: A delivery system
   - 130: A hypotube / a hypotube shaft / a tube
   - 132: An atraumatic tip at a distal end of the hypotube
   - 140: An embolic protection device
   - 142: A deflection-filter of the embolic protection device
   - 144: A device-tail/connection mechanism of the embolic protection device
   - 146: A connector of the embolic protection device
   - 150: A guidewire
Figures 2a and 2b illustrate deployed embolic protection assemblies according to the invention in use.
   - 201: An embolic protection assembly according to the invention
   - 211: A handle
   - 220: A delivery system
   - 223: Forced arching of delivery system
   - 230: A hypotube / a hypotube shaft / a tube
   - 232: An atraumatic tip at a distal end of the hypotube
   - 240: An embolic protection device
   - 242: A deflection-filter
   - 244: A device-tail/connection mechanism
   - 250: A guidewire
   - 251: Distal end of a guidewire formed as a pigtail
   - 255: Valve
   - 260: A locking mechanism
Figure 3 illustrates schematically an example of a basic locking mechanism 370 according to the invention.
   - 350: A guidewire
   - 370: A basic locking mechanism according to the invention
   - 371: A screw
   - 372: A fixed lock unit, clamping box/lockable passage
   - 375: A first end of the locking mechanism
   - 376: A second end of the locking mechanism
Figure 4 illustrates schematically an example of a combined locking and dampening mechanism 480 according to a further aspect of the invention.
   - 450: A guidewire
   - 471: A screw
   - 480: A dampening locking mechanism according to the invention
   - 482: A non-fixed lock unit, clamping box/lockable passage
   - 483: A long narrow hole, a slot/slit for the screw to slide in
   - 485: A first end of the locking mechanism
   - 486: A second end of the locking mechanism
   - 487: A first spring or spring function, linear or progressive
   - 488: A second spring or spring function, linear or progressive
Figure 5 illustrates schematically an example of a combined locking and dampening mechanism with a controllable release 590 according to a further aspect of the invention.
   - 550: A guidewire
   - 587: A first spring or spring function, linear or progressive
   - 588: A second spring or spring function, linear or progressive
   - 590: A releasable dampening locking mechanism according to the invention
   - 591: External, suitably tubular, extension of the second end 596
   - 592: A non-fixed lock unit, clamping box/lockable passage
   - 595: A first end of the locking mechanism
   - 596: A second end of the locking mechanism
   - 597: Internal tubular tapered extension of the first end 595
   - 598: Internal tubular tapered extension of the second end 596
   - 599: Threads between the first end 595 and the extension 591 of the second end 596

## Claims

1. An introducer assembly for minimally invasive deployment of a medical device in a vessel, the introducer assembly comprising:
a guidewire (250, 450, 550);
a handle (110, 211) comprising a locking mechanism ( 480, 590) for locking the guidewire (250, 450, 550) relative said handle (110, 211);
a medical device, wherein said medical device is an embolic protection device (140, 240);
a tube (130, 230) attached to the handle (110, 211);
wherein the introducer assembly is configured for the guidewire (250, 450, 550) to be drawn through the tube (130, 230), and the locking mechanism (480, 590) is configured in the introducer assembly, such that, the guidewire (250, 450, 550) drawn through the tube (130, 230) is also drawn through the locking mechanism (480, 590),
and wherein the locking mechanism (480, 590) is in a first state configured to positionally lock the guidewire (250, 450, 550) that is drawn through the locking mechanism (480, 590) and in a second state the locking mechanism (480, 590) is configured to allow the guidewire (250, 450, 550) to positionally move; and
**characterized in that** the locking mechanism (480, 590) includes at least one spring (487, 488, 587, 588) arranged to provide limited relative movement of the guidewire (250, 450, 550) relative the handle (110, 211) while in a locked state of the locking mechanism (480, 590).

2. The introducer assembly according to claim 1, wherein said embolic protection device (140, 240) includes a filter (242) or a deflector unit such as a deflection filter (242).

3. The introducer assembly according to claim 1, wherein the tube (130, 230) is directly or indirectly attached to the embolic protection device.

4. The introducer assembly according to claim 2, wherein a pressure on said deflection filter (242) by said tube (130, 230) is controllable by a position of said guidewire (250, 450, 550) relative said handle (110, 211) to control a position of said deflection filter (242) in said vessel.

5. The introducer assembly according to any previous claim, wherein the locking mechanism (480, 590) comprises a lock unit (482, 592) and wherein the guidewire is positionally locked in relation to the lock unit.

6. The introducer assembly according to claim 5, wherein the lock unit (482, 592) is configured to clamp the guidewire (250, 450, 550) in the first state.

7. The introducer assembly according to any of claims 1 to 6, wherein the locking mechanism (480, 590) is further configured in the first state to subject the guidewire (250, 450, 550) to a predetermined friction so that as long as the guidewire (250, 450, 550) is subjected to a force in relation to the locking mechanism (480, 590) that is below a first predetermined force level, it is positionally locked.

8. The introducer assembly according to any of claims 1 to 7, wherein the locking mechanism (480, 590) is further configured in the first state to change to the second state if the guidewire (250, 450, 550) is subjected to a force in relation to the locking mechanism (480, 590) that is above a second predetermined force level.

9. The introducer assembly according to claim 1, wherein the at least one spring (487, 488, 587, 588) are linear or progressive.

## Patentansprüche

1. Eine Einführanordnung zum minimalinvasiven Einsatz einer medizinischen Vorrichtung in einem Gefäß, wobei die Einführanordnung Folgendes umfasst:
einen Führungsdraht (250, 450, 550);
einen Griff (110, 211), der einen Verriegelungsmechanismus (480, 590) zum Verriegeln des Führungsdrahts (250, 450, 550) relativ zu dem Griff (110, 211) umfasst;
eine medizinische Vorrichtung, wobei es sich bei der medizinischen Vorrichtung um eine Embolieschutzvorrichtung (140, 240) handelt;
einen am Griff10, 211) befestigten Schlauch (130, 230);
wobei die Einführanordnung so ausgelegt ist, dass der Führungsdraht (250, 450, 550) durch den Schlauch (130, 230) gezogen werden kann, und der Verriegelungsmechanismus (480, 590) in der Einführanordnung so angeordnet ist, dass der Führungsdraht (250, 450, 550), der durch den Schlauch (130, 230) gezogen wird, auch durch den Verriegelungsmechanismus (480, 590) gezogen wird, und wobei der Verriegelungsmechanismus (480, 590) in einem ersten Zustand so ausgelegt ist, dass er den Führungsdraht (250, 450, 550), der durch den Verriegelungsmechanismus (480, 590) gezogen wird, positionsmäßig arretiert, und der Verriegelungsmechanismus (480, 590) in einem zweiten Zustand so ausgelegt ist, dass er eine Positionsbewegung des Führungsdrahts (250, 450, 550) zulässt; und
**dadurch gekennzeichnet, dass** der Verriegelungsmechanismus (480, 590) mindestens eine Feder (487, 488, 587, 588) umfasst, die so angeordnet ist, dass sie im verriegelten Zustand des Verriegelungsmechanismus (480, 590) eine begrenzte Relativbewegung des Führungsdrahts (250, 450, 550) relativ zum Griff (110, 211) ermöglicht.

2. Die Einführanordnung nach Anspruch 1, wobei die Embolieschutzvorrichtung (140, 240) einen Filter (242) oder eine Deflektoreinheit, wie beispielsweise einen Deflektorfilter (242), umfasst.

3. Die Einführvorrichtung nach Anspruch 1, wobei der Schlauch (130, 230) direkt oder indirekt an der Embolieschutzvorrichtung befestigt ist.

4. Die Einführanordnung nach Anspruch 2, wobei ein Druck auf den Deflektorfilter (242) durch den Schlauch (130, 230) durch eine Position des Führungsdrahts (250, 450, 550) relativ zum Griff (110, 211) steuerbar ist, um eine Position des Deflektorfilters (242) in dem Gefäß zu steuern.

5. Die Einführanordnung nach einem der vorstehenden Ansprüche, wobei der Verriegelungsmechanismus (480, 590) eine Verriegelungseinheit (482, 592) umfasst und wobei der Führungsdraht in Bezug auf die Verriegelungseinheit positionsfest arretiert ist.

6. Die Einführanordnung nach Anspruch 5, wobei die Verriegelungseinheit (482, 592) so ausgebildet ist, dass sie den Führungsdraht (250, 450, 550) im ersten Zustand festklemmt.

7. Die Einführanordnung nach einem der Ansprüche 1 bis 6, wobei der Verriegelungsmechanismus (480, 590) im ersten Zustand ferner so ausgelegt ist, dass er den Führungsdraht (250, 450, 550) einer vorbestimmten Reibung aussetzt, sodass, solange der Führungsdraht (250, 450, 550) einer Kraft in Bezug auf den Verriegelungsmechanismus (480, 590) ausgesetzt ist, die unter einem ersten vorgegebenen Kraftniveau liegt, er positionsmäßig verriegelt ist.

8. Die Einführanordnung nach einem der Ansprüche 1 bis 7, wobei der Verriegelungsmechanismus (480, 590) im ersten Zustand ferner so ausgelegt ist, dass er in den zweiten Zustand wechselt, wenn auf den Führungsdraht (250, 450, 550) in Bezug auf den Verriegelungsmechanismus (480, 590) eine Kraft einwirkt, die über einem zweiten vorgegebenen Kraftniveau liegt.

9. Die Einführanordnung nach Anspruch 1, wobei die mindestens eine Feder (487, 488, 587, 588) linear oder progressiv ist.

## Revendications

1. Ensemble introducteur pour déploiement minimalement invasif d'un dispositif médical dans un vaisseau, l'ensemble introducteur comprenant :
un fil de guidage (250, 450, 550) ;
une poignée (110, 211) comprenant un mécanisme de verrouillage (480, 590) destiné à verrouiller le fil de guidage (250, 450, 550) par rapport à ladite poignée (110, 211) ;
un dispositif médical, où ledit dispositif médical est un dispositif de protection embolique (140, 240) ;
un tube (130, 230) attaché à la poignée (110, 211) ;
où l'ensemble introducteur est configuré pour permettre de tirer le fil de guidage (250, 450, 550) à travers le tube (130, 230), et le mécanisme de verrouillage (480, 590) est configuré dans l'ensemble introducteur de telle sorte que le fil de guidage (250, 450, 550) tiré à travers le tube (130, 230) est également tiré à travers le mécanisme de verrouillage (480, 590), et où le mécanisme de verrouillage (480, 590) est, dans un premier état, configuré pour verrouiller en position le fil de guidage (250, 450, 550) qui est tiré à travers le mécanisme de verrouillage (480, 590) et, dans un second état, le mécanisme de verrouillage (480, 590) est configuré pour permettre au fil de guidage (250, 450, 550) de changer de position ; et
**caractérisé en ce que** le mécanisme de verrouillage (480, 590) inclut au moins un ressort (487, 488, 587, 588) agencé pour assurer un déplacement relatif limité du fil de guidage (250, 450, 550) par rapport à la poignée (110, 211) lorsque le mécanisme de verrouillage (480, 590) se trouve dans un état verrouillé.

2. Ensemble introducteur selon la revendication 1, dans lequel ledit dispositif de protection embolique (140, 240) inclut un filtre (242) ou une unité déviatrice telle qu'un filtre de déviation (242).

3. Ensemble introducteur selon la revendication 1, dans lequel le tube (130, 230) est directement ou indirectement attaché au dispositif de protection embolique.

4. Ensemble introducteur selon la revendication 2, dans lequel une pression sur ledit filtre de déviation (242) exercée par ledit tube (130, 230) peut être contrôlée par une position dudit fil de guidage (250, 450, 550) par rapport à ladite poignée (110, 211) pour contrôler une position dudit filtre de déviation (242) dans ledit vaisseau.

5. Ensemble introducteur selon l'une quelconque des revendications précédentes, dans lequel le mécanisme de verrouillage (480, 590) comprend une unité de verrou (482, 592) et où le fil de guidage est verrouillé en position par rapport à l'unité de verrou.

6. Ensemble introducteur selon la revendication 5, dans lequel l'unité de verrou (482, 592) est configurée pour fixer le fil de guidage (250, 450, 550) dans le premier état.

7. Ensemble introducteur selon l'une quelconque des revendications 1 à 6, dans lequel le mécanisme de verrouillage (480, 590) est en outre configuré dans le premier état pour soumettre le fil de guidage (250, 450, 550) à un frottement prédéterminé de telle sorte que, tant que le fil de guidage (250, 450, 550) est soumis à une force par rapport au mécanisme de verrouillage (480, 590) qui est inférieure à un premier niveau de force prédéterminé, il est verrouillé en position.

8. Ensemble introducteur selon l'une quelconque des revendications 1 à 7, dans lequel le mécanisme de verrouillage (480, 590) est en outre configuré dans le premier état pour passer au second état si le fil de guidage (250, 450, 550) est soumis à une force par rapport au mécanisme de verrouillage (480, 590) qui est supérieure à un second niveau de force prédéterminé.

9. Ensemble introducteur selon la revendication 1, dans lequel les au moins un ressorts (487, 488, 587, 588) sont linéaires ou progressifs.
